# EUROPEAN PATENT SPECIFICATION

(11) **EP 2 705 763 B1**
(45) Date of publication and mention of the grant of the patent: **27.12.2017**
(21) Application number: 12780064.7
(22) Date of filing: 24.04.2012
(51) Int. Cl.: A61K 31/164, A61P 1/04, A61P 35/00, C12N 1/16, C12P 19/44, A61K 47/26, A61K 47/46, A61K 36/06, A23L 33/145

(54) **METHOD FOR UTILIZING EXTRACTION RESIDUE OF YEAST EXTRACT**
VERFAHREN ZUR VERWENDUNG VON EXTRAKTIONSRÜCKSTÄNDEN EINES HEFEEXTRAKTS
PROCÉDÉ D'UTILISATION DE RÉSIDUS D'EXTRACTION D'UN EXTRAIT DE LEVURE

(30) Priority: 02.05.2011 JP 2011102895; 06.04.2012 JP 2012087010
(43) Date of publication of application: 12.03.2014
(73) Proprietor: KOHJIN Life Sciences Co., Ltd., Tokyo 100-0006 (JP)
(72) Inventor: SATO, Toshiya, Tokyo 1000006 (JP); NAKAGAWA, Tomohiro, Saiki-shi Oita 876-8580 (JP); KAJI, Naoto, Saiki-shi Oita 876-8580 (JP)
(74) Representative: Müller-Boré & Partner Patentanwälte PartG mbB
(86) International application number: PCT/JP2012/060948
(87) International publication number: WO 2012/150683

(56) References cited:
- JP-A- H04 248 968
- JP-A- 2002 281 936
- JP-A- 2003 000 197
- JP-A- 2003 221 592
- JP-A- 2004 161 618
- JP-A- 2005 185 126
- JP-A- 2005 187 392
- JP-A- 2010 022 217
- JP-A- 2010 022 218
- J. RUPCIC ET AL: "Cerebrosides of Candida lipolytica yeast", APPLIED MICROBIOLOGY AND BIOTECHNOLOGY, vol. 64, no. 3, 1 April 2004 (2004-04-01), pages 416-420, XP055130412, ISSN: 0175-7598, DOI: 10.1007/s00253-003-1533-y

## Description

The present invention relates to a method of producing a glucosylceramide composition using an organic solvent to extract the glucosylceramide composition from a yeast residue after extraction of yeast extract, wherein the yeast is *Candida utilis,* the glucosylceramide composition being applicable to a functional food, a cosmetic product, a medicinal product and the like.

Glucosylceramide is a type of glycosphingolipid, in which one molecule of glucose is bonded to a ceramide backbone composed of an amide-linked sphingoid base and fatty acid. Glucosylceramide, which is widely distributed in animals, plants, and microorganisms, is effective in improving skin functions or preventing large intestine cancer when taken as a supplement. Thus, glucosylceramide has recently been drawing attention as a material for health foods, cosmetic products, and medicinal products.

Glucosylceramide was originally extracted from bovine brain. Since the outbreak of BSE, however, safer plant materials have been used. Currently, glucosylceramide is extracted from various materials, including konjac fly powder, rice bran, wheat bran, and corn germ, and is commercially sold as a ceramide-containing extract. However, concerns about use of such plant materials include quality susceptible to weather or storage conditions after harvest, potential supply instability and price fluctuation depending on crop yields, and enormous cost of material procurement due to a minute amount of glucosylceramide contained in raw materials, which are thus required in large amounts.

Against this backdrop, methods have been studied for producing glucosylceramide using yeast, which is more stable in quality and supply, as a raw material. Prospective materials studied include baker's yeasts and brewer's yeasts (Saccharomyces cerevisiae), which are industrially mass-produced, and yeast cell bodies of beer residue. These Saccharomyces cerevisiae, however, cannot produce glucosylceramide. Thus, studies have been conducted on other Saccharomyces, Zygosaccharomyces, Kluyveromyces yeasts, Pseudozyma, Candida, and Pichia yeasts, and methods of production have been proposed. These yeasts can be mass-produced at one site in a closed system, and thus quality control and material procurement are considered to be easy compared to plant materials.

In this context, JP 4-248968 A describes an edible extraction residue of yeast extract and a method for producing the same. Further, Rupćič and Marić (Rupćič, J. and Marić, V.; Applied Microbial and Cell Physiology, 64(3); 2004; pp. 416-420) describes the isolation and characterization of cerebrosides from *Candida lipolytica.* Furthermore, JP 2005-185126 A describes a method for the large-scale production of cerebroside from fungal raw material, as well as respective cerebroside compositions.

However, while a source of nutrients needs to be introduced in a large amount for culturing yeast, glucosylceramide content is still low in yeast cell bodies, similar to plants. Thus, problems exist, including high production cost and generation of a large amount of industrial waste. In addition, glucosylceramide compositions produced from yeast in a conventional method contain a large amount of contaminants, including sterol glycosides, which are difficult to remove. Furthermore, currently commercially available glucosylceramide-containing extract is mostly used for food, and thus yeast used as a raw material should have been eaten by human beings and should belong to safe species. Thus, production of glucosylceramide from yeast is difficult and poses many problems.

### [Patent Literature]

Patent Literature 1: Japanese Patent Laid-Open Publication No. 2004-89047
Patent Literature 2: Japanese Patent Laid-Open Publication No. 2005-312372
Patent Literature 3: Japanese Patent Laid-Open Publication No. 2005-187392
Patent Literature 4: Japanese Patent Laid-Open Publication No. 2006-55070
Patent Literature 5: Japanese Patent Laid-Open Publication No. 2008-245607
Patent Literature 6: Japanese Patent Laid-Open Publication No. 2010-22217
Patent Literature 7: Japanese Patent Laid-Open Publication No. 2010-22218
Patent Literature 8: Japanese Patent Laid-Open Publication No. 2002-519070
Patent Literature 9: Japanese Patent Laid-Open Publication No. 2003-221592

An objective of the present invention is to produce a high-quality glucosylceramide composition from a raw material that has been eaten by human beings and is stable in quality, supply, and price.

The inventors of the present invention conducted research to solve the above-described problems associated with use of plant materials and yeast materials. As a result, the inventors have found that a glucosylceramide-containing composition has few contaminants when the glucosylceramide-containing composition is extracted from a raw material of a yeast residue using an organic solvent, the yeast residue being generated in a yeast extract production process after extraction of the extract currently mass-produced for food use. Thus, the inventors have reached the present invention.

Yeast used as the raw material for the yeast extract is Torula yeast (Candida utilis) since its safety is confirmed as it has been eaten by human beings and it contains a large amount of glucosylceramide.

The present invention relates to a method of producing a glucosylceramide composition using an organic solvent to extract the glucosylceramide composition from a yeast residue after extraction of yeast extract, wherein the yeast is *Candida utilis.*

According to the present invention, a high-quality glucosylceramide composition having few contaminants can be produced from a residue of Torula yeast, which has been eaten by human beings and thus known to be safe. The glucosylceramide composition is particularly excellent for having few contaminants, such as sterol glycosides, which are included in large quantity in plant materials and are difficult to remove.

Yeast extract of Torula yeast is suitable as a flavoring agent and is mass-produced. With effective use of its residue, the method of the present invention is also very advantageous in view of cost and reduction in industrial waste. Unlike a case of agricultural crops used as raw materials, there is little risk of supply instability, price fluctuation, and quality variation.

Yeast used in the present invention is *Candida utilis,* which is generally called Torula yeast. A method of culturing the yeast is either batch culture or continuous culture. A culture medium for culturing may have a general culture medium composition. Specifically, examples of a carbon source may include glucose, acetic acid, ethanol, glycerol, molasses, and spent sulfite liquor. Examples of a nitrogen source may include urea, ammonia, ammonium sulfate, ammonium chloride, and nitrate. As sources of phosphoric acid, potassium, and magnesium, normal industrial materials may be used, including calcium superphosphate, ammonium phosphate, potassium chloride, potassium hydroxide, magnesium sulfate, and magnesium chloride. Furthermore, inorganic salts, such as zinc, copper, manganese, and iron ions, may be added. Additionally, vitamins, amino acids, and nucleic acid-related substances may be added; or organic materials, such as casein, yeast extract, meat extract, and peptone, may be added. The culture temperature is 21 to 37°C, preferably 25 to 34°C; and pH is 3.0 to 8.0, with 3.5 to 7.0 being particularly preferred. Productivity of amino acids or nucleic acids fluctuates depending on culture conditions. Thus, the conditions are defined to meet product specifications of a target yeast extract.

Extract is extracted after culturing a cell body. In the present application, yeast from which extract has been extracted is referred to as a yeast residue. A method of extraction is not particularly limited. Generally, extraction can be performed in an autolysis method, a hot water extraction method, an enzymatic extraction method, an acid or alkaline extraction method, or a combination of these methods.

To extract yeast extract in autolysis, stirring is performed for four hours at a temperature of 55°C, for example. In the enzymatic extraction method, stirring and extraction is performed with a cell wall lytic enzyme, protease, or the like. In the acid extraction method, extraction is performed after acidification with a sulfuric acid or the like. In the alkaline extraction method, extraction is performed after alkalization. Alternatively, a combination of autolysis (stirring for four hours at 55°C, for example) and then enzymatic extraction may be employed. A water-soluble fraction extracted and separated as above is yeast extract, which is suitably used as a flavoring agent. What remains after the yeast extract is separated and removed is a yeast residue.

Through the extraction of the extract in yeast above, proteins and nucleic acids in the cell body are extracted and removed and some fat-soluble contaminants, such as sterol glycosides, are removed. Thus, a yeast residue enriched with glucosylceramide is produced. In the present application, glucosylceramide is extracted from such a yeast residue, and then a glucosylceramide composition from which contaminants, such as sterol glycosides, are removed is produced without a special treatment. In the present application, qualitative analysis by TLC and quantitative analysis by HPLC-ELSD are performed on contaminants and glucosylceramide.

After the extraction of the extract, the yeast residue is collected by centrifugal separation. Then, the residue is suspended in distilled water and centrifugally separated repeatedly for cleaning. The cleaned residue can be freeze-dried or hot-air dried when necessary. The produced yeast residue is a Torula yeast-derived glucosylceramide raw material.

Then, glucosylceramide is extracted from the raw material above. A method of extraction is not limited, but a method of extraction for food use is preferred. Specifically, a method disclosed in Japanese Patent Laid-Open Publication No. 2002-281936 can be employed for refining. For instance, an organic solvent is used in the extraction from the yeast residue as a raw material. In view of food use, the organic solvent is preferably an ethanol, acetone, or hexane, and an ethanol is particularly preferred. In addition, water or a surfactant can be added to the organic solvent to improve extraction efficiency. An amount of the solvent for extraction is preferably 1 to 30 times the amount of the raw material.

More specifically, a 90 to 100% ethanol is used in an amount twice that of a Torula yeast-derived yeast residue, and stirring is performed for 1 to 24 hours to extract glucosylceramide. After extraction, extraction liquid is collected by centrifugal separation, and then is concentrated and freeze-dried or hot-air dried to yield a glucosylceramide composition.

The glucosylceramide composition can be further refined, when necessary, to produce a composition having a high glucosylceramide content or to produce glucosylceramide. A known method can be employed as a method of refinement, including silica gel or ion-exchange resin column cleanup, for example.

The glucosylceramide composition and its refined materials, which are the composition having a high glucosylceramide content and the glucosylceramide, according to the present invention are extracted from highly safe yeast, and thus can be used as a health food, a functional food such as a supplement, a cosmetic product, or a medicinal product.

### Embodiment

The present invention is described in detail in embodiments below. The present invention, however, is not limited to the embodiments.

### (TLC Conditions)

As pretreatment, 20 mg of a glucosylceramide composition specimen was dissolved in 2 ml of 0.4N KOH EtOH, and then was incubated for two hours at a temperature of 37°C. After the incubation, 2 ml of 0.4N HCl EtOH was added for neutralization, and then after centrifugal separation 4 µl of a supernatant was provided for TLC. TLC was performed with a silica gel plate (Merck Silica Gel 60; a layer thickness of 0.25 mm) and developed with chloroform:ethanol:water = 65:16:2 (capacity ratio). After the development, the silica gel plate was dried, and then sprayed with an anisaldehyde sulfuric acid test solution and heated for color development.

### (HPLC-ELSD Conditions)

As pretreatment, 20 mg of a glucosylceramide composition specimen was dissolved in 2 ml of 0.4N KOH EtOH, and then was incubated for two hours at a temperature of 37°C. After the incubation, 2 ml of 0.4N HCl EtOH was added for neutralization, and then salts were removed by a filter to be provided for HPLC. GL Science Inertsil 100A was used as a column. Glucosylceramide was detected with an evaporative light scattering detector (Shimadzu ELSD-LTII). Chloroform and a gradient of methanol:water = 95:5 (capacity ratio) were used as a dissolution medium. A column temperature was 35°C. A flow velocity was 1 ml/min. A drift-tube temperature was 40°C. A nitrogen gas pressure was 350 kPa.

### (Culture of Yeast)

Candida utilis CS 7529 strain (FERM BP-1656) was seed-cultured in advance in a YPD culture medium (1% yeast extract, 2% polypeptone, 2% glucose) in a conical flask, and then 1 to 2% thereof was inoculated in an 18 L culture medium in a 30 L fermentation tank. A culture medium composition was 4% glucose, 0.3% ammonium dihydrogen-tetraoxophosphate, 0.161% ammonium sulfate, 0.137% potassium chloride, 0.08% magnesium sulfate, 1.6 ppm copper sulfate, 14 ppm iron sulfate, 16 ppm manganese sulfate, and 14 ppm zinc sulfate. Culture conditions were a pH of 4.0, a culture temperature of 30°C, an aeration rate of 1 vvm, and a stirring rate of 600 rpm. Ammonia was added to control the pH. After a cell body was cultured for 16 hours, a culture solution was collected, and then the cell body was collected by centrifugal separation to yield 180 g of wet yeast cell body.

### (Extraction of Yeast Extract)

The wet yeast cell body obtained after the cell body culture was suspended in distilled water and centrifugally separated repeatedly for cleaning. After the cleaning, the wet cell body was suspended in distilled water again in that state, or was freeze-dried or hot-air dried to produce a dry cell body. Thereafter, the cell body was suspended in distilled water, and then extract was extracted under the conditions below.

Autolysis: The pH was controlled to 5.0 with 1N HCl, and then stirring was performed for four hours at a temperature of 55°C.

Enzymatic extraction: The pH was controlled to 7 with 1N NaOH, and then stirring and extraction was performed with a cell wall lytic enzyme (Amano Enzyme Tunicase) or protease for four hours at a temperature of 55°C.

Acid extraction: The pH was controlled to 2 or less with 1N sulfuric acid, and then stirring and extraction was performed for two minutes at a temperature of 65°C.

Alkaline extraction: The pH was controlled to 13 with 2N NaOH, and then stirring and extraction was performed for 20 minutes at a temperature of 70°C.

### Embodiment 1 (Autolysis)

Sixty g of a dry cell body of Torula yeast, which was cleaned after cell body culture in the method above, was suspended in 1 L of distilled water and was controlled to a pH of 5.0 with 1N HCl. Extract was extracted by stirring for four hours at a temperature of 55°C. A yeast residue was collected by centrifugal separation, and then was cleaned by three courses of suspension in distilled water and centrifugal separation. Then, the cleaned yeast residue was vacuum dried to yield 10.9 g of a dry yeast residue, which was provided as a Torula yeast-derived glucosylceramide raw material. The entire amount of the produced dry yeast residue was suspended in an amount of a 90% ethanol twice that of the residue, and then was stirred for 12 hours at a temperature of 60°C to extract glucosylceramide. Extraction liquid was collected by centrifugal separation, and was then mixed with cleaning liquid that cleaned the residue three times with an ethanol and was condensed. Thus, 0.6 g of an extracted material was produced as a glucosylceramide composition. Based on analysis by HPLC-ELSD, the glucosylceramide composition contained 2.0% glucosylceramide. Furthermore, from analysis by TLC, only a glucosylceramide spot was identified proximate to an Rf value of 0.42, and sterol glycosides, which are contaminants, were not observed. The analysis results demonstrated that the glucosylceramide content in the Torula yeast-derived glucosylceramide raw material was 0.11 %.

### Embodiment 2 (Enzymatic Extraction)

Sixty g of a dry cell body of Torula yeast, which was cleaned and hot-air dried after cell body culture, was suspended in 1 L of distilled water and was controlled to a pH of 7.0 with 1N NaOH. Then, yeast extract was extracted with protease for four hours at a temperature of 55°C. After the extraction, a yeast residue was collected by centrifugal separation, and then was cleaned by three courses of suspension in distilled water and centrifugal separation. Then, the cleaned yeast residue was vacuum dried to yield 12.8 g of a dry yeast residue, which was provided as a Torula yeast-derived glucosylceramide raw material. The entire amount of the produced dry yeast residue was suspended in an amount of a 90% ethanol twice that of the residue, and then was stirred for 12 hours at a temperature of 60°C to extract glucosylceramide. Extraction liquid was collected by centrifugal separation, and was then mixed with cleaning liquid that cleaned the yeast residue three times with an ethanol and was condensed. Thus, 0.8 g of an extracted material was produced as a glucosylceramide-containing composition. Based on analysis by HPLC-ELSD, the glucosylceramide-containing composition contained 1.6% glucosylceramide. Furthermore, from analysis by TLC, only a glucosylceramide spot was identified proximate to an Rf value of 0.42, and sterol glycosides, which are contaminants, were not observed. The analysis results demonstrated that the glucosylceramide content in the Torula yeast-derived glucosylceramide raw material was 0.10%.

### Embodiment 3 (Acid Extraction)

Sixty g of a dry cell body of Torula yeast, which was cleaned and hot-air dried after cell body culture, was suspended in 1 L of distilled water and was controlled to a pH of 2.0 with 2N NaOH sulfuric acid. Then, extract was extracted for two minutes at a temperature of 65°C. After the extraction, a yeast residue was collected by centrifugal separation, and then was cleaned by three courses of suspension in distilled water and centrifugal separation. Then, the cleaned yeast residue was vacuum dried to yield 11.3 g of a dry yeast residue, which was provided as a Torula yeast-derived glucosylceramide raw material. The entire amount of the produced dry yeast residue was suspended in an amount of a 90% ethanol twice that of the residue, and then was stirred for 12 hours at a temperature of 60°C to extract glucosylceramide. Extraction liquid was collected by centrifugal separation, and was then mixed with cleaning liquid that cleaned the yeast residue three times with an ethanol and was condensed. Thus, 0.6 g of an extracted material was produced as a glucosylceramide-containing composition. Based on analysis by HPLC-ELSD, the glucosylceramide-containing composition contained 1.9% glucosylceramide. Furthermore, from analysis by TLC, only a glucosylceramide spot was identified proximate to an Rf value of 0.42, and sterol glycosides, which are contaminants, were not observed. The analysis results demonstrated that the glucosylceramide content in the raw material was 0.10%.

### Embodiment 4 (Alkaline Extraction)

Sixty g of a dry cell body of Torula yeast, which was cleaned and hot-air dried after cell body culture, was suspended in 1 L of distilled water and was controlled to a pH of 13.0 with 2N NaOH. Then, extract was extracted for 30 minutes at a temperature of 70°C. After the extraction, a yeast residue was collected by centrifugal separation, and then was cleaned by three courses of suspension in distilled water and centrifugal separation. Then, the cleaned yeast residue was vacuum dried to yield 12.3 g of a dry yeast residue, which was provided as a Torula yeast-derived glucosylceramide raw material. The entire amount of the produced dry yeast residue was suspended in an amount of a 90% ethanol twice that of the residue, and then was stirred for 12 hours at a temperature of 60°C to extract glucosylceramide. Extraction liquid was collected by centrifugal separation, and was then mixed with cleaning liquid that cleaned the yeast residue three times with an ethanol and was condensed. Thus, 0.7 g of an extracted material was produced as a glucosylceramide-containing composition. Based on analysis by HPLC-ELSD, the glucosylceramide-containing composition contained 2.1% glucosylceramide. Furthermore, from analysis by TLC, only a glucosylceramide spot was identified proximate to an Rf value of 0.42, and sterol glycosides, which are contaminants, were not observed. The analysis results demonstrated that the glucosylceramide content in the raw material was 0.12%.

### Comparative Example 1

Twenty g of a dry cell body of Torula yeast, which was cleaned and hot-air dried after cell body culture, was suspended in an amount of a 90% ethanol twice that of the cell body, and then was stirred for 10 hours at a temperature of 60°C to extract glucosylceramide. Extraction liquid was collected by centrifugal separation, and was then mixed with cleaning liquid that cleaned a solid content three times with an ethanol and was condensed. Thus, 0.9 g of an extracted material was produced as a glucosylceramide-containing composition. Based on analysis by HPLC-ELSD, the glucosylceramide-containing composition contained 1.4% glucosylceramide. Furthermore, from analysis by TLC, a glucosylceramide spot was identified proximate to an Rf value of 0.42. In addition, sterol glycosides, which are contaminants, were observed proximate to an Rf value of 0.48, and a lipid spot and others were also identified. The analysis results demonstrated that the glucosylceramide content in the raw material, which was the Torula yeast cell body, was 0.06%.

The glucosylceramide composition having few contaminants, produced according to the present invention, is applicable as a material for a functional food or a cosmetic product in that state or after further refinement. In addition, provided that medicinal benefits are recognized in the future, the glucosylceramide composition is also applicable as a raw material for a medicinal product.

## Claims

1. A method of producing a glucosylceramide composition using an organic solvent to extract the glucosylceramide composition from a yeast residue after extraction of yeast extract, wherein the yeast is *Candida utilis.*

## Patentansprüche

1. Verfahren zur Herstellung einer Glucosylceramidzusammensetzung unter Verwendung eines organischen Lösungsmittels, um die Glucosylceramidzusammensetzung aus einem Heferückstand nach Extraktion eines Hefeextrakts zu extrahieren, wobei die Hefe *Cadida utilis* ist.

## Revendications

1. Une méthode de production d'une composition de glucosylcéramide qui utilise un solvant organique pour extraire la composition de glucosylcéramide à partir d'un résidu de levure après extraction d'un extrait de levure, où la levure est *Candida utilis.*
